# EUROPEAN PATENT APPLICATION

(11) **EP 3 751 524 A1**
(43) Date of publication of application: **16.12.2020**
(21) Application number: 19180004.4
(22) Date of filing: 13.06.2019
(51) Int. Cl.: G06T 19/20

(54) **METHOD, COMPUTER PROGRAM PRODUCT AND COMPUTER SYSTEM FOR PROVIDING AN APPROXIMATE IMAGE**

(71) Applicant: RaySearch Laboratories AB, 103 65 Stockholm (SE)
(72) Inventor: Eriksson, Kjell, 74651 Bålsta (SE); Weistrand, Ola, 14169 Huddinge (SE); Svensson, Stina, 11161 Stockholm (SE); Andersson, Sebastian, 118 24 Stockholm (SE)

(57) **Abstract**

An approximate image of a patient can be provided by the following method:
- Obtaining a model of the contour of a patient's body or a part of the patient's body, based on a surface scan of the patient
- Setting at least one interior density or image intensity value for at least a part of the interior of the contour to provide an approximation of the density of the patient's interior
- Setting an exterior density or image intensity value for the region outside of the contour,
- Creating an approximate image based on the model of the contour, and the density or image intensity values.

The interior density or intensity may be set to a uniform image intensity value for the whole image or one or more regions, such as organs or bone, may be distinguished by different density of image intensity values.

## Description

### Technical Field

The present invention relates to a method, a computer program product and a computer system for providing an estimated image of a patient for use in medical applications.

### Background

Various types of imaging for medical purposes are widely used, for example, for planning surgery or other types of treatment, such as radiotherapy treatment. In some medical applications an image of a large portion of the patient's body is required. Depending on the imaging method this takes time and requires the patient to be still in more or less comfortable positions.

One known method for providing high-quality data is computer tomography, CT. It is known to obtain a full-body CT scan but this takes time and also involves exposing the person being imaged to an amount of radiation, which is generally undesirable. CT images are often used for radiotherapy treatment planning that includes dose computation, including forward or inverse planning techniques.

One such medical application requiring images if an accurate dose distribution is to be computed is treatment planning for total body irradiation (TBI), which is used in preparations for bone marrow transplants and blood transplants. For such treatments an approximative planning of the dose is often sufficient and they have traditionally been planned based on very limited data, such as the position of one point in the patient's body and/or the body size. For example, Int. J. of Rd one Bio Phys, Volume 84, issue 3, Supplement, Page S864, states that "conventional Total Body Irradiation (TBI) planning requires that the whole body receives within +/- 10% of the prescribed dose where the dose calculation is performed for a point midline at the umbilicus. Koch et al: Total Body Irradiation using dynamic arc delivery, Int J Radiat Oncol Biol Phys, 2016; 96:E640, discloses a method where the calculations are based on body size, without any further anatomical information. The results of planning based on such input data are sometimes unsatisfactory. The planning method disclosed by Koch et al is based on a library of pre-commissioned dose dynamic fields, or arcs, which are scaled using a measure of the patient size.

TBI today is performed at a long distance from the patient, typically 4 meters or more, to cover the full body. The greater distance means that each beam will cover a larger area of the patient so that fewer beams, possibly only one beam, is required. This results in a large field and significant uncertainties. Alternatively, a moveable couch is used, and the radiation is provided to one portion of the body at the time. Suggested methods are based on e.g. template beam arrangements. If a full body CT is not acquired such methods will suffer from large uncertainties in dose computation since the treatment plan is not based on a 3D image of the patient. In particular, there will be uncertainties in dose at the beam junctions (often called field junctions) due to patient position uncertainties in relation to the beam geometry, i.e. in the junction between two beams covering adjacent volumes of the patient.

Another field of application is planning of surgery. For some types of surgery the patient is imaged lying on the back and then turned on the side and tilted or bent. The treatment position can then be very different from the imaging position. The positions of internal organs may be difficult to predict in this case and obtaining a full CT scan in this position is not feasible. It is desirable to be able to obtain a reliable approximation of the internal organs in the treatment position in such cases.

### Summary

It is an object of the present invention to enable the provision of an approximative image of a patient with a higher precision than previous approximations, without the need for a detailed image, such as a CT image, of the patient. As explained above, such an approximative image can be used for planning of various medical procedures such as radiotherapy or surgery.

This object is achieved according to the present invention by a computer implemented method of providing an approximate 3D image of a patient, comprising the following steps:
- Obtaining a model of the contour of a patient's body or a part of the patient's body, based on a surface scan of the patient
- Setting at least one interior value, which may be a density and/or image intensity value for at least a part of the interior of the contour to provide an approximation of the density of the patient's interior
- Setting an exterior value, which may be a density and/or image intensity value for the region outside of the contour different from the at least one interior density value,
- Creating an approximate image based on the model of the contour, the at least one interior value and the exterior value.

The use of density value or image intensity value is selected depending on the image modality. In parts of this document, the discussion is based on density values. It should be understood that for some image modalities image intensity values could or should be used instead. The resulting image is an approximation of the interior of the patient which is adapted to the patient's outer contour and possibly to other data related to the patient's interior. The approximative image may be used for various medical applications, including radiotherapy treatment planning or planning of surgery.

In the simplest embodiment, one interior intensity or density value for the whole part of the interior of the contour, for example an interior density value representing water. In all of the embodiments, the density outside of the contour is preferably set to a suitable density, such as the density of air.

In other embodiments, a first interior density value may be set for a first part of the interior of the contour and a second interior density value may be set for a second part of the inside of the contour. In this way, regions having different density values may be distinguished in the image to create a more correct image. For example, the method may comprise the step of approximating a shape and position of at least a first region of interest in the interior of the patient, within the contour and setting the density value for the first region of interest to the first interior density value, to point out the particular region of interest, which may be a target, such as a tumor, or an organ at risk, or another region that should be identified for some reason.

It is also possible to combine the single density value with data about the position and extent of the bone structure within the patient, obtained, for example, from X-ray imaging, such as fluoroscopy. The bone structure information is preferably in 3D but useful data may also be obtained from 2D images. From a 2D X-ray or a 2D MR image a contour of the bone structure may be obtained that may help determine the direction of deformation. If two or more such images are provided, preferably orthogonal to each other, precision is improved. In this way, additional data may be provided in shorter time and with less radiation dose to the patient than if a CT image is to be provided. In the 2D images it is also possible to identify other types of tissue, such as lung tissue which may then be used in the approximate image. Including information regarding the bone structure is suitable because it is less detailed and therefore less complex than a complete set of information about all organs and tissues in the patient while at the same time providing important data such as the position of the spinal cord. Also, the density of bone differs significantly from the density of other tissues and organs within the patient so that this type of data has a great impact on the treatment planning.

In some preferred embodiments, the step of approximating a shape and position of said at least first region comprises providing a CT image of an interior of a patient and approximating the shape and position of the first region of interest based on the shape and position of a corresponding region of interest in the image. The step of approximating typically comprises registering the CT image to the contour by deformable registration but it may also be based on, for example, machine learning. This will provide very detailed interior information inside the contour, which is useful for some applications but may be unnecessary for other applications requiring less detail.

The CT image may be an image of the patient or may be based on a number of images of other patients, or on a previous image taken of the patient.

The method according to the invention therefore provides an approximative technique suitable for use, for example, with TBI that can be delivered in a smaller setting. This is because the approximate image data enables more exact planning than planning without any image. It also makes it possible to obtain a more correct planning for field junctions. At the same time, the approximate image data can be obtained without a full-body CT image of the patient, which saves time and resources and avoids unnecessary radiation to the patient.

The use of surface scanning apparatus is becoming more widely spread in radiotherapy treatment units, where they are used for ensuring correct positioning of patients for delivery of treatment fractions. According to the invention, such apparatus can be used for the purpose of providing surface images of the patient's outline in 3D, which may be filled with approximate image data of the patient's interior. The approximate image data may be provided in different ways, with different levels of accuracy. The level of accuracy may be determined based on the intended use of the approximate image. This is useful, in particular for providing full body images, or images of a large part of the patient's body with approximate interior.

The images resulting from the method may also be used for other purposes, such as planning of palliative radiotherapy treatment or other situations where an approximative dose is desired or at least sufficiently good and/or it is important to get a result within a short timeframe. In particular in the case of palliative treatment, not having to move the patient to obtain a CT scan can improve their comfort significantly. A suitable CT image, such as a planning image taken of the patient at an earlier point in time, or an atlas image representative of the patient, may be used to provide interior data. The CT image is registered to the contour, preferably by deformable registration, to adjust the interior of the patient to the new contour.

The method is suitable for any treatment modality, such as photons, protons, electrons or ions. It may be applied for any treatment technique, including VMAT, SMLC, DMLC, 3DCRT, Conformal Arc, static Arcs, PBS. It may also be used for any treatment planning technique, including forward planning, inverse planning, MCO, knowledge based planning, and automatic planning. The method may also be used for planning of other types of medical treatment, including surgery,

The method according to the invention may be combined with robust optimization to account for uncertainties, such as field junctions and/or for the uncertainty caused by the use of estimated images. If an approximation of the position of organs, or a synthetic CT image is included, the robust optimization may also be used to account for uncertainties in the internal patient geometry and/or changes that have occurred after the planning image was taken. Robust optimization can also be used with respect to any other uncertainty, such as patient setup, density and/or organ motion.

The invention also relates to a method of planning a medical procedure including performing method according to any one of the embodiments discussed above to provide an approximate 3D image of a patient and planning the treatment based on the approximate 3D image. The medical procedure may be any type of medical procedure, for example a radiation therapy treatment or a surgical procedure and the planning of the treatment may be based on the 3D image in any suitable way. A number of such image-based planning methods are known in the art. For radiation therapy planning, the image may be used for computer-based treatment planning in any manner known in the art. For other procedures, such as surgery, the planning may involve a medical practitioner deciding on surgical steps based on the positions of important tissues or organs within the patient.

Preferably, since the 3D image is an approximate representation of the patient, the treatment planning is robust treatment planning with respect to the uncertainties of the approximate 3D image. For example, the radiation therapy planning method may comprise providing at least two different approximate 3D images of the patient using the above method and using the at least two approximate 3D images to ensure that the treatment plan will yield a satisfactory result for each of the at least two approximate 3D images. This is useful for embodiments in which at least a first and a second interior value is used, for example, for a bone structure and surrounding tissue. It is particularly suitable when the step of approximating a shape and position of said at least first region comprises providing a CT image of an interior of a patient and approximating the shape and position of the first region of interest based on the shape and position of a corresponding region of interest in the image.

To achieve robust planning, the difference between the at least two approximate images may be achieved in different ways. For example, the position and/or shape of one or more regions of interest may be varied between the two images of the interior. These regions of interests could for example be assigned with an interior value, or be used as controlling structures to guide the deformations so that deformations with the same patient surface, but different internal structures are obtained. Another possibility is to use images of historical patients with different internal geometries.

The method also relates to a computer program product and a computer system for providing the approximate patient image.

### Brief Description of the Drawings

The invention will be described in more detail in the following, by way of example and with reference to the appended drawings, in which
Figures 1 is a flow chart of a first embodiment of the method according to invention
Figure 2 is a flow chart of a second embodiment of the method according to invention
Figure 3 is a flow chart of a method of computing an external contour
Figure 4 illustrates a third embodiment of the method
Figures 5a and 5b illustrate a fourth embodiment of the method
Figure 6 shows an example of a general dose delivery system that may also be used for treatment planning

### Detailed Description

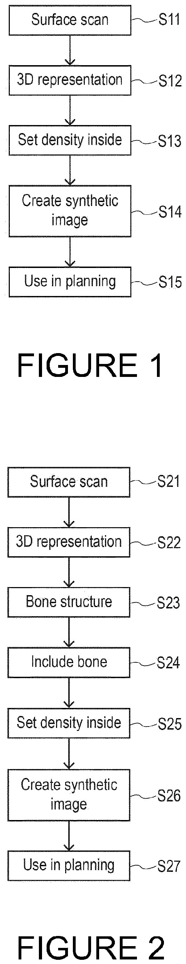

Figure 1 is a flow chart of a first embodiment of the invention. This method is simple to implement and useful in cases where a low level of accuracy is sufficient.

In a first step S11, a surface scan of a patient is provided. The surface scan may be obtained directly from the scanning of a patient or may be a previous scanning result retrieved from a memory and may cover the whole body or a part of the body.

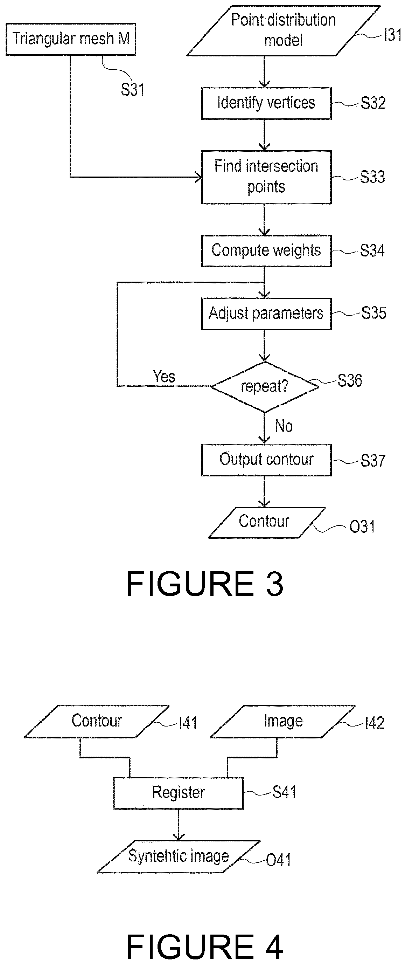

In a second step S12, a 3D representation of the contour of the patient is provided based on the surface scan, for example, as discussed in connection with Figure 3. In a third step S13, the density inside the contour is set to a first suitable value, for example, the density of water, and the density outside of the contour is set to a second suitable value, for example the density of air. Based on this, in step S14 a synthetic image of a body having the same outline as the patient, filled with a homogenous substance and surrounded by air is created. Water is suitable because the density is close to the average density of the body. This provides a fast and simple way of obtaining a simplified image of the patient's body, which may be used for applications in which details about the patient's interior, such as the positions of tissues or organs, are of less importance.

In a fifth step S15, the synthetic image is used for the purpose of planning a medical procedure, such as radiotherapy treatment planning or surgery planning.

Two surface scanners that are suitable for providing the surface scan are available from C-RAD and VisionRT, respectively. These scanners provide tools for monitoring a patient's position for planning and delivery of radiation. Its use is also foreseen for other applications, for example for monitoring a patient's position during certain types of surgery, and for monitoring respiratory activity.

Figure 2 is a flow chart of a second embodiment of the invention, in which data related to the bone structure of the patient is included.

In a first step S21, a surface scan of a patient is provided and in a second step S22, a 3D representation of the contour of the patient is provided in the same manner as described for Figure 1.

In a third step S23 information regarding the patient's bone structure is obtained. This may be obtained using fluoroscopy or another suitable X-ray imaging method, as discussed above.

In a fourth step S24, the bone structure information obtained in step S23 is superimposed on the image of the body in the right position corresponding to the actual position of the skeleton within the patient. In a fifth step S25, the density inside the contour is set. In this case, however, this step also uses the information regarding the patient's bone structure. The density of the areas corresponding to bone structure is set to a value corresponding to a density for bone and the density of the rest of the interior of the contour is set to a suitable value representative of general tissue, such as water, as for Figure 1. The density outside of the contour is preferably set to a suitable value, for example the density of air. In this way, a synthetic image of a body having the same outline as the patient is created but in this case the interior is approximated with respect to size and position of skeleton.

In a sixth step S26, the synthetic image is created and may be used for the purpose of planning a medical procedure, such as radiotherapy treatment or surgery. In this case, the synthetic image includes all or part of the patient's bone structure distinguished from the rest of the patient's interior by means of differently set density values.

The output data from surface scanning devices may have different formats and normally some processing may be required to obtain a complete external contour based on the data.

An external contour may be computed from a surface scan of the patient in question, by applying it to a general model of a patient's surface, which may be the patient's own surface obtained at an earlier stage, or another suitable model of a body surface. Figure 3 discloses one possible method of computing a complete external contour from a surface scan:

In a first step S31 points from the surface scan are converted to a triangular mesh M by means of 3D surface reconstruction, if this is not already provided by the scanning device.

If the surface scan does not cover the whole body, a Point Distribution Model (PDM) is preferably used as input data I31 together with the triangular mesh obtained in step S31 to provide approximate surface data outside of the area covered by the scan. The inventive method preferably uses a full body scan. The PDM may be obtained in any suitable way, including being trained from surfaces from a patient population or only consisting of one triangulated mesh with no shape components. Such a triangulated mesh suitable for use in the current procedure could be the external contour of the current patient computed from a CT scan or an MR scan.

Using the mesh M and the PDM as input data, the PDM is adapted to M using an iterative procedure as follows:

In a step S32, vertices are identified on the PDM. In a step S33, for each vertex identified on the PDM in step S32, a search is performed along a finite interval in the normal direction of PDM to find intersection points with M.

In a step S34, a weight is computed for each intersection point identified in step S33, based on the angle between the normal of PDM and the normal of M at the intersection point.

Step S35 is performed iteratively and involves adjusting translation, rotation and shape parameters of PDM to adapt PDM to M. In this step, deformations of PDM that allow deviation from the shape model defined in PDM may also be allowed. In this case, a regularization measure for the deformation is used.

The adjustments made in step S35 should be repeated as many times as is found feasible, typically until a stopping criterion is met. The stopping criterion may be that the adjustments should be performed a certain number of times, or that a certain quality criterion is met. In step S36, it is determined, whether a further iteration of step S35 is to be performed. When it is determined that no further iterations should be performed, the contour O31 as adjusted is output in a step S37, for use in planning.

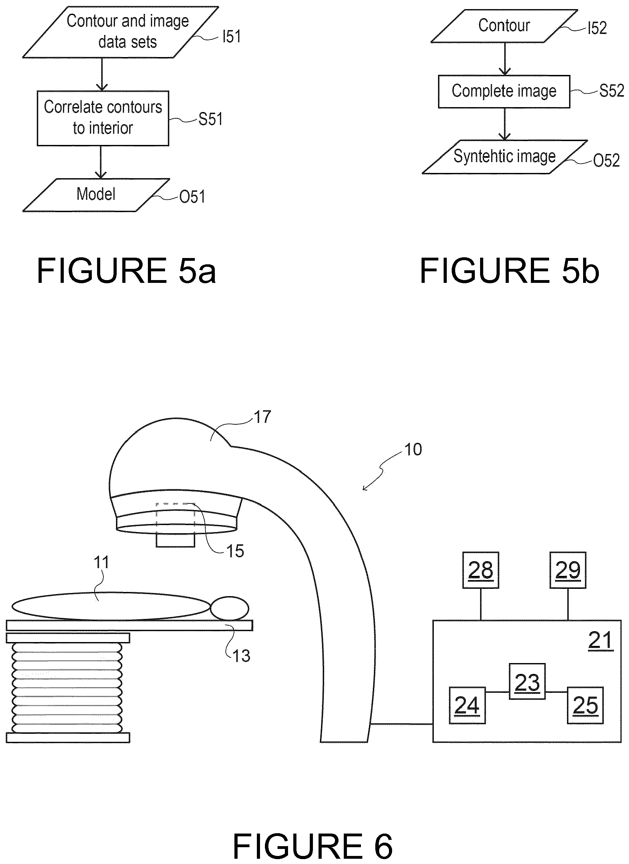

An estimated image of the inside of the whole contour, or of a part of the contour, can be obtained in different ways, by means of deformable registration or machine learning, as will be discussed in connection with Figures 4, 5a and 5b. These procedures will result in an image that is closer to an actual image of the patient than the resulting images from the procedures in Figures 1 and 2, with less time, discomfort and dose to the patient than providing a CT scan.

With reference to Figure 4, a synthetic CT image may be created in the following way, using the external contour I41 of the current patient and a previously acquired CT scan 142 as input data. The external contour may be obtained using a surface scanner and if necessary post-processing the data, for example, as discussed in connection with Figure 3. The CT scan I42 is a full-body scan or covers at least the part of the patient that should be examined, and may be obtained in any suitable way. It is preferably an earlier CT scan of the patient, but may alternatively be another representative image, for example, a full-body scan of another patient, or an average image of several patients. Such an average image can be obtained from a number of images of different patients by deforming the images and fusing the result. Methods of doing this are well known in the art. A library of suitable CT images for different types of patients may be provided, divided by shape, size, gender, age or other parameters and the most suitable CT image to be used in the procedure may be chosen manually or automatically.

In step S41 a deformable registration algorithm is applied to deform the CT scan I42 to the external contour I41. The bone structure may be used as a controlling region of interest ROI. The result of the deformation is a synthetic CT image O41 which is an approximation of the interior of the current patient. This synthetic image can be used for treatment planning for example for providing a radiotherapy treatment plan. If appropriate for the planning, the density of the couch is also added to the image. An MR image may be used instead of the CT image. In this case, the MR image may be deformed to the external contour and then converted to CT format, or first converted to CT format and the resulting CT image be deformed to the external contour. For applications in which the density information is not needed, an MR image may be used without conversion to CT format.

The whole interior of the contour may be approximated in this way, or only a portion of the patient or one or more specific organs or regions of interest may be included and the rest may be filled with a suitable homogenous approximative density value such as the density of water.

A similar procedure to the one in Figure 4 may be used to obtain an approximate image of a patient with segmented regions such as organs instead of a full CT image. In this case, a segmented image is used as the previously acquired image I42. Registration is performed in essentially the same way as with a full CT image.

In alternative embodiments to those discussed in connection with Figure 4, machine learning is used to predict a synthetic CT image, a synthetic MR image, or a synthetic segmented image of the interior of the external contour. In this case a deep learning method is used to compute either an image with segmented regions inside or a full synthetic CT image. The network is trained as illustrated in Figure 5a: As input data a number of data sets I51 providing contour data of a number of patients together with information about the interior of the same patient are provided. In a similar way as for Figure 4, the information may be grouped according to size, shape, age and/or gender. The contour data may be obtained from a surface scan, or from an image, such as a CT image, also providing the interior information. In a step S51, a deep learning method is used to find correlations between the shape of the contour and the position of the respective organs within the contour and to train the network to predict the position of organs within a given patient contour. The output is a function for how to estimate the interior of patients in dependence of their exterior contours.

Figure 5b is a flow chart of how to use the machine learning model to provide data about the interior of a specific contour obtained from a patient. The contour is provided as input data 152, for example in the manner discussed in connection with Figure 3. The deep learning model is then applied to the contour to compute a segmentation, or a full CT image is then computed, which will be an approximation of the patient's interior.

The information about the interior of each patient may be a full CT image or a segmented image indicating only the position and shape of one or more organs or other regions of interest. Hence, the result may be a contour with approximated segmentations of one or more organs inside, or a full synthetic CT scan. As discussed for Figure 4, this may be performed for the full contour, or only a region or portion of the patient may be segmented or provided with CT image data.

Figure 6 is an overview of a system 10 for radiotherapy treatment and/or treatment planning. As will be understood, such systems may be designed in any suitable way and the design shown in Figure 6 is only an example. A patient 1 is positioned on a treatment couch 3. The system comprises an imaging/treatment unit having a radiation source 5 mounted in a gantry 7 for emitting radiation towards the patient positioned on the couch 3. Typically, the couch 3 and the gantry 7 are movable in several dimensions relative to each other, to provide radiation to the patient as flexibly and correctly as possible. These parts and their functions are well known to the skilled person. A number of passive devices provided to shape the beam laterally and in depth are typically present and will be not be discussed in more detail here. In this example the system also comprises an optical scanner for providing data related to the outer contour of the patient and enabling the generation of a contour image of the whole or part of the patient's body. The system also comprises a computer 21 which may be used for radiotherapy treatment planning and/or for controlling radiotherapy treatment. As will be understood, the computer 21 may be a separate unit not connected to the imaging/treatment unit.

The computer 21 comprises a processor 23, a data memory 24, and a program memory 25. Preferably, one or more user input means 28, 29 are also present, in the form of a keyboard, a mouse, a joystick, voice recognition means or any other available user input means. The user input means may also be arranged to receive data from an external memory unit.

The data memory 24 comprises clinical data and/or other information used to obtain a treatment plan, including the contour data provided by the contour scanner. The data memory 24 also comprises one or more patient images to be used in treatment planning according to embodiments of the invention. The nature of these patient images, and how they may be obtained, has been discussed above. The program memory 25 holds at least one computer program arranged to cause the processor to perform a method according to any of the Figures 1 - 4, 5a and/or 5b. The program memory 25 also holds a computer program arranged to make the computer perform the method steps discussed in connection with Figure 4 or 5 and/or a computer program arranged to make the computer control the radiotherapy treatment of a patient.

As will be understood, the data memory 24 and the program memory 25 are shown and discussed only schematically. There may be several data memory units, each holding one or more different types of data, or one data memory holding all data in a suitably structured way, and the same holds for the program memories. One or more memories may also be stored on other computers. For example, the computer may be arranged to perform only one of the methods, there being another computer for performing the optimization.

## Claims

1. A computer-based method of providing an approximate 3D image of a patient, comprising the following steps:
- Obtaining a model of the contour of a patient's body or a part of the patient's body, based on a surface scan of the patient
- Setting at least one interior value which may be a density and/or image intensity value for at least a part of the interior of the contour to provide an approximation of the density of the patient's interior
- Setting an exterior value which may be a density and/or image intensity value for the region outside of the contour different from the at least one interior density value,
- Creating an approximate image based on the model of the contour, the at least one interior value and the exterior value.

2. A method according to claim 1, wherein one interior value for the whole part of the interior of the contour, for example an interior value representing water.

3. A method according to claim 1, further comprising the step of setting a first interior value for a first part of the interior of the contour and a second interior value for a second part of the inside of the contour,

4. A method according to claim 3, further comprising the step of approximating a shape and position of at least a first region of interest in the interior of the patient, within the contour and setting the first interior value for the first region of interest to the first interior value.

5. A method according to claim 4, further comprising the step of providing bone information relating to the position of the patient's skeleton and estimating a position and shape of a bone region in the interior of the contour based on the bone information and setting the first or second interior value in a region corresponding to the bone region to a density representative of bone.

6. A method according to claim 4 or 5, wherein the step of approximating a shape and position of said at least first region comprises providing a 3D image, which may be a CT image or an MR image, of an interior of a patient and approximating the shape and position of the first region of interest based on the shape and position of a corresponding region of interest in the image.

7. A method according to claim 6, wherein the step of approximating comprises registering the 3D image to the contour by deformable registration

8. A method according to claim 6 or 7, wherein the 3D image is based on a number of images of patients, or on a previous image taken of the patient.

9. A method according to any one of the preceding claims, wherein the exterior value is set to a value representing air.

10. A method of planning a medical procedure, such as radiation therapy or surgery, said method including performing the method according to any one of the claims 1 - 9 to provide an approximate 3D image of a patient and planning the medical procedure based on the approximate 3D image.

11. A method according to claim 10, wherein the medical procedure is planned with respect to the uncertainties of the approximate 3D image.

12. A method according to claim 11, comprising performing the method according to any one of the claims 1 - 9 at least two times, to provide at least two approximate 3D images of the patient and use the at least two approximate 3D images in planning the treatment, to ensure that the treatment will yield a satisfactory result for each of the at least two approximate 3D images.

13. A computer program product, preferably stored in a non-transitory storage means (55) which, when run in a computer will cause the computer to perform the method of any one of the claims 1-9 and/or the planning method of any of the claims 10 - 12.

14. A computer system (21) for providing an approximate image of a patient, the system comprising processing means (23), said computer system having a program memory (25) having stored therein a computer program product according to claim 10 in such a way that the computer program product, when executed, will control the processing means (23) to perform the dose planning.

15. A computer system according to claim 14, further comprising a contour scanning device arranged to provide contour data representing the contour or the patient's body and the processor is arranged to calculate the model of the contour based on the contour data.
